(19) Europäisches Patentamt
European Patent Office
Office européen des brevets

(11) Publication number: **0 064 306**
**B1**

(12) # EUROPEAN PATENT SPECIFICATION

(45) Date of publication of patent specification: **21.05.86**

(21) Application number: **82200461.0**

(22) Date of filing: **15.04.82**

(51) Int. Cl.4: **C 07 D 307/12,** C 07 D 307/14, C 07 D 407/04, C 07 D 405/14, C 07 D 405/12, A 01 N 43/08, A 01 N 43/26, A 01 N 43/40 // C07D317/30, C07D317/20, C07C69/738

(54) **Substituted tetrahydrofuran herbicides.**

(30) Priority: **01.05.81 GB 8113522**

(43) Date of publication of application: **10.11.82 Bulletin 82/45**

(45) Publication of the grant of the patent: **21.05.86 Bulletin 86/21**

(84) Designated Contracting States: **BE CH DE FR GB IT LI NL**

(56) References cited:
**EP-A-0 000 002**
**EP-A-0 013 581**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Orr, Alexander Ferguson**
**6 Amber Close Teynham**
**Sittingbourne Kent (GB)**
Inventor: **Clifford, David Ronald**
**66 Northwood Drive**
**Sittingbourne Kent (GB)**

(74) Representative: **Hunter, Keith Roger Ian et al**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

The present invention relates to substituted tetrahydrofuran derivatives, a process for their preparation, herbicide compositions containing them and a method of controlling unwanted plant growth using them.

Certain tetrahydrofuran derivatives are known to be active herbicides, for example, see German Offenlegungsschrift No. 2749974 and European Patent Application No. 0000002. It has now been found that certain novel tetrahydrofuran derivatives also have useful herbicidal properties.

The present invention provides a tetrahydrofuran derivative of the general formula:—

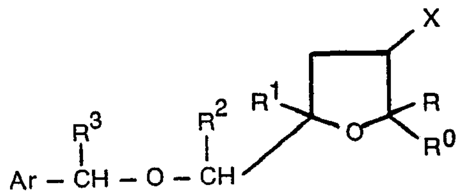

(I)

wherein each of $R^0$, R, $R^1$, $R^2$, and $R^3$ independently represents a hydrogen atom or an alkyl group or $R^0$ and R together represent an alkylene group; X represents one of the groups

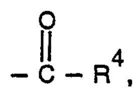 , 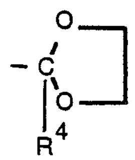 , 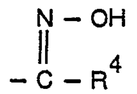 or 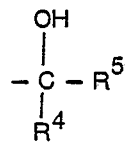

wherein $R^4$ represents a hydrogen atom or an optionally substituted aryl or alkyl group and $R^5$ represents a hydrogen atom, an alkyl group, an alkenyl group or an alkynyl group; and Ar represents an optionally substituted fully unsaturated ring having 5 or 6 atoms in the ring of which one is a nitrogen atom and the remainder are carbon atoms, or the N-oxide or an acid addition salt thereof, or Ar represents an optionally substituted phenyl group.

Suitable substituents which may be present in an optionally substituted aliphatic group include halogen atoms, alkoxy, alkylthio, cyano, carboxy, alkoxycarbonyl, amino and cycloalkyl groups, optionally substituted phenyl and phenoxy groups, and groups of the formula —OA in which A represents a hydrogen atom or an acyl group derived from a carboxylic or a substituted carbamic acid. Suitable substituents which may be present in an optionally substituted aryl, especially phenyl, group include halogen atoms, hydroxy, amino, cyano and nitro groups, and optionally substituted alkyl, alkenyl, alkynyl, alkoxy, alkylthio, alkoxycarbonyl, aryl and aryloxy groups.

An aryl group and in particular a phenyl group is preferably unsubstituted or substituted by up to 3 of the same or different substituents given above. Most preferably it is unsubstituted, monosubstituted or disubstituted. Preferred substituents are selected from halogen atoms, and nitro, cyano, alkyl, haloalkyl, alkoxy, haloalkoxy, alkylthio, aryl and aryloxy groups. Typically the substituents are selected from halogen atoms and alkyl, alkoxy, alkylthio, aryl and aryloxy groups.

A heterocyclic group Ar may be substituted by one or more of the substituents given above for a phenyl group; preferably however it is unsubstituted or substituted by one or more alkyl groups or halogen atoms; most preferably such a group is unsubstituted.

Preferably $R^0$ and R are the same and each represents a hydrogen atom or an alkyl group of up to 6 carbon atoms; preferably $R^1$ represents an alkyl group of up to 6 carbon atoms; preferably $R^2$ and $R^3$ each represent a hydrogen atom; preferably $R^4$ represents an alkyl group of up to 6 carbon atoms; and preferably $R^5$ represents a hydrogen atom or an alkyl, alkenyl or alkynyl group of up to 6 carbon atoms.

More preferably $R^0$ and R are the same and each represents an alkyl group of up to 4 carbon atoms; $R^1$ represents an alkyl group of up to 4 carbon atoms; $R^2$ and $R^3$ each represent a hydrogen atom; $R^4$ represents an alkyl group of up to 4 carbon atoms; and $R^5$ represents a hydrogen atom or an alkyl or alkynyl group of up to 4 carbon atoms.

As noted above, the Ar substituent in formula I may represent an optionally substituted fully unsaturated ring having 5 or 6 atoms in the ring of which one is a nitrogen atom. Suitable heterocyclic groups in this regard include optionally substituted pyridyl, pyrrolyl and azacyclopentadiene groups. These heterocyclic groups are preferably bonded to the rest of the molecule through a carbon atom and the nitrogen atom in the ring is preferably adjacent to this carbon atom. Preferably Ar represents a pyridyl, especially a 2-pyridyl, group or an optionally substituted phenyl group, especially an unsubstituted phenyl group or a phenyl group substituted, preferably in the 2-position, with an alkyl group of up to 3 carbon atoms, or by one or more fluorine and/or chlorine atoms preferably in the 2-position by a chlorine atom, a fluorine atom or in the 2 and 6 positions with a chlorine atom or a fluorine atom or a mixture thereof.

Most preferred compounds according to formula I are those wherein each of $R^0$ and R represents a methyl group, $R^1$ represents an ethyl group, each of $R^2$ and $R^3$ represents a hydrogen atom, $R^4$ represents a methyl group, $R^5$ represents a hydrogen atom, and Ar represents a phenyl group, a 2-chlorophenyl group, a 2-fluorophenyl group or a 2-pyridyl group.

The compounds of the general formula I exist in the form of optical isomers, and may also exist as geometric isomers, depending on the substituents present in the molecule. It should be understood that the present invention includes all such isomers and mixtures thereof.

The invention also provides a process for the preparation of a compound according to the invention in which a tetrahydrofuran derivative II

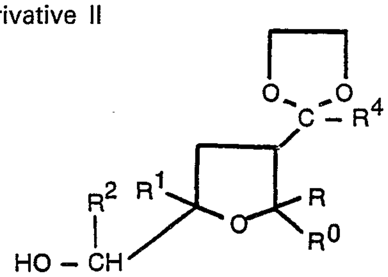

(II)

is converted into an alkali metal or alkaline earth salt thereof, and reacted with a compound of the general formula

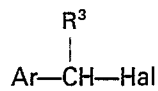

(III)

in which Hal represents a halogen atom and $R^3$ and Ar have the meanings given for the general formula I; and optionally converting the resulting compound of the formula I in which X represents a

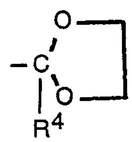

group, into any other compound of the general formula I.

The compound of general formula II may be converted into a salt thereof by reaction with a base. Alkali or alkaline earth metal hydroxides, alkoxides or hydrides are suitable bases. The alcohol may be converted into its salt prior to admixing it with the compound of formula III, or the salt may be formed *in situ* by admixing the compounds of the formulae II and III in the presence of a base. Any suitable solvent may be used for the reaction, for example an aromatic hydrocarbon, for example benzene or toluene. The reaction may for example be carried out at a temperature in the range of from 50 to 150°C. Conveniently, the reaction is carried out at the reflux temperature of the solvent used.

The tetrahydrofuran derivative II may be prepared by subjecting a compound of the general formula IV

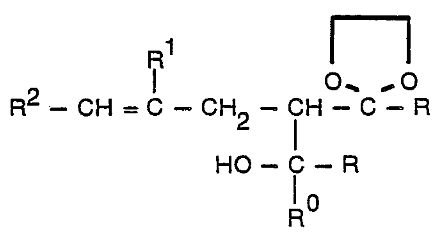

(IV)

to ring closure by reaction with an electrophilic epoxidizing agent.

Suitable electrophilic epoxidizing agents include hydrogen peroxide, alkali metal peroxides or hypohalites, metal perborates, peroxyacetyl nitrate and silver oxide. Especially suitable electrophilic epoxidising agents are peroxyacids, for example aliphatic peroxyacids such as peroxyacetic acid or peroxyformic acid, or, preferably, aromatic peroxyacids such as unsubstituted or substituted peroxybenzoic acid. Especially suitable are halogen-substituted peroxybenzoic acids, for example acids in which the phenyl ring is substituted by one or two chlorine and/or bromine atoms. Meta-chloroperoxybenzoic acid is especially suitable.

The epoxidation is suitably carried out in the presence of an inert solvent, for example a hydrocarbon, chlorinated hydrocarbon, ether or ester, such as benzene, toluene, methylene chloride, carbon tetrachloride, diethyl ether or ethyl acetate. Mixtures of solvents may be suitable.

The epoxidation is preferably carried out at a temperature in the range of from −10°C to 80°C,

especially 0 to 20°C. It may in some cases be convenient to carry out the reaction at the reflux temperature of the solvent used.

The molar ratio of the compound of the general formula IV and the electrophilic epoxidising agent is not of critical importance. Preferably the compound of the general formula IV and the electrophilic epoxidising agent are mixed in approximately equimolar quantities, or a slight excess of the epoxidising agent is used. Preferably the molar ratio of the compound of the general formula I to the electrophilic epoxidising agent is in the range of from 1:1 to 1:2 especially 1:1 to 1:1.5. Useful yields can however be obtained using a molar ratio of up to 1:10 or higher.

The compounds of the general formula IV may be prepared by methods analogous to methods known in the art.

Compounds of the general formula I in which X represents a

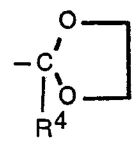

group are convenient sources of other tetrahydrofuran derivatives in accordance with general formula I since the dioxolane functional group can be readily converted to the corresponding carbonyl moiety, that is compounds of general formula I wherein X represents a

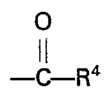

group, by treatment with an acid. Compounds of general formula I wherein X represents a

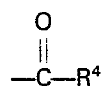

group may then be converted to other compounds of general formula I by methods analogous to methods known in the art. For example, the oxime derivatives, that is compounds wherein X represents

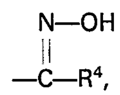

may be obtained by treating the carbonyl compound with hydroxylamine hydrochloride in the presence of an organic base such as pyridine. Further, the carbonyl group may be converted to the corresponding alcohol by reaction with a suitable reducing agent, for example, lithium aluminium hydride in ether solvent. The Grignard reaction may be employed to convert the carbonyl group into an alcohol while introducing the appropriate alkyl, alkenyl or alkynyl substituents onto the carbonyl carbon atom, that is compounds according to general formula I wherein X represents a

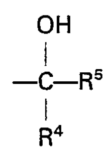

group.

The compounds of general formula I exhibit herbicidal activity. Therefore the invention further provides a herbicidal composition which comprises a compound of the formula I together with a suitable carrier. The invention also provides a method of combating undesired plant growth at a locus, which comprises applying to the locus a compound or a composition according to the invention.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; synthetic hydrated silicon oxides and synthetic calcium or

aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. In general, such compositions may contain from 0.5 to 95% w of the active ingredient with the remainder being adjuvants conventionally employed in such compositions, that is carriers and/or surface active agents. Wettable powders usually contain 25, 50 and 75% w of active ingredient and usually contain in addition to solid inert carrier, 3—10% w of a dispersing agent and, where necessary, 0—10% w of stabiliser(s) and/or other additives such as penetrants of stickers. Dusts are usually formulated as a dust concentrate having a similar composition to that of a wettable powder but without a dispersant, and are diluted in the field with further solid carrier to give a composition usually containing ½—10% w of active ingredient. Granules are usually prepared to have a size between 10 and 100 BS mesh (1.676—0.152 mm), and may be manufactured by agglomeration or impregnation techniques. Generally, granules will contain ½—25% w active ingredient and 0—10% w of additives such as stabilisers, slow release modifiers and binding agents. Emulsifiable concentrates usually contain, in addition to a solvent and, when necessary, co-solvent, 10—50% w/v active ingredient, 2—20% w/v emulsifiers and 0—20% w/v of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thixotropic agents, 0—10% w of other additives such as defoamers, corrosion inhibitors, stabilisers, penetrants and stickers, and water or an organic liquid in which the active ingredient is substantially organic liquid in which the active ingredient is substantially insoluble; certain organic solids or inorganic salts may be present dissolved in the formulation to assist in preventing sedimentation or as antifreeze agents for water.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

The composition of the invention may also contain other ingredients, for example, other compounds possessing herbicidal, insecticidal or fungicidal properties.

The following Examples illustrate the invention. NMR values are values relative to tetramethylsilane in $CDCl_3$.

Example 1

2-Ethyl-2-benzyloxymethyl-4-[2-(2-methyl-1,3-dioxolanyl)]-5,5-dimethyl oxolane

(a) Sodium (6.8 g) was dissolved in methanol (500 ml) and methyl acetoacetate (35.67 g) was added over a 10 minute period. After stirring for 15 minutes 2-bromoethyl-but-1-ene (50 g) was added over a period of 30 minutes and the mixture was refluxed and stirred for a further 2 hours. The mixture was then poured onto brine and extracted 4 times with ether. The extract was dried using magnesium sulphate and the ether was evaporated off. Distillation of the residue under vacuum gave 36.7 g, corresponding to a

64.7% yield of 2 - ethyl - 4 - methylcarbonyl - 4 - methoxycarbonyl - but - 1 - ene, boiling point 110—118°C at a pressure of 8 mm Hg.

(b) The product from (a) (34 g) was combined with ethylene glycol (12.6 g), benzene solvent (550 ml) and a catalytic amount of toluene sulphonic acid (100 mg) and the mixture was refluxed for 158 hours with periodic additions of small amounts of ethylene glycol and catalyst to promote the reaction. At the end of the reaction period, the mixture was washed with sodium bicarbonate solution and dried with magnesium sulphate. Distillation of the product under vacuum afforded 29.8 g, corresponding to a yield of 70.7% of 2 - ethyl - 4 - [2 - (2 - methyl - 1,3 - dioxolanyl)] - 4 - methoxycarbonyl - but - 1 - ene, boiling point 127—132°C at a pressure of 8 mm Hg.

(c) Magnesium (6 g) was initially mixed with diethyl ether (50 ml) under nitrogen and to this mixture was added a solution of methyl iodide (25 g) in diethyl ether (50 ml). The mixture was stirred until the magnesium had dissolved at which time the ester product from (b) (25 g) in diethyl ether solvent (150 ml) was added and the mixture was refluxed for 1 hour. Subsequently, the mixture was washed with aqueous ammonium chloride solution and twice with ether. The ether extracts were combined, washed with brine and dried using magnesium sulphate. The ether solvent was evaporated off and the residue was distilled under vacuum and the fractions boiling at 81—86°C at 0.5 mm Hg and 86—91°C at 0.5 mm Hg were collected and chromatographed on a silica column using 4% acetone/petrol as eluant. Evaporation of the solvent afforded 14 g, corresponding to a 56% yield of 2 - ethyl - 4 - [2(2 - methyl - 1,3 - dioxolanyl)] - 4 - (2 - hydroxyisopropyl) - but - 1 - ene. Its NMR spectrum was as follows:

δ1.0 (12H, M), 1.5—2.3 (5H, M), 3.9 (4H, s), 4.3 (1H, br), 4.7 (2H, br s).

(d) The olefinic alcohol prepared in (d) (20 g) was dissolved in methylene chloride (50 ml) and added to *m*-chloroperbenzoic acid (21.76 g of 85% pure material) in methylene chloride (120 ml). The mixture was stirred for 17 hours after which it was distilled under vacuum to afford 10.66 g, corresponding to a 49.7% yield of 2 - ethyl - 2 - hydroxymethyl - 4 - [2 - (2 - methyl - 1,3 - dioxolanyl)] - 5,5 - dimethyl oxalane, boiling point 112—122°C at a pressure of 0.7 mm Hg. Its NMR spectrum was as follows:

δ0.9 (3H, t), 1.2 (M, 9H), 1.3—2.5 (6H, M), 3.4 (2H, br d), 3.9 (4H br s).

(e) The oxolane prepared in (d) (10.66 g) in toluene (ml) was added slowly to a stirred mixture of sodium hydride (1.37 g) in toluene (100 ml) under nitrogen. The mixture was refluxed for 1 hour at which time a solution of benzyl bromide (9.75 g) in toluene (10 ml) was added slowly. This mixture was refluxed for 18 hours after which it was washed with brine and extracted with ether. The ether extract was washed with brine and dried over magnesium sulphate. The ether solvent was evaporated off and the residue was chromatographed using a silica column and 3% acetone/petrol as eluant. Evaporation of the solvent afforded 14.7 g, corresponding to a 64.1% yield of 2 - ethyl - 2 - benzyloxymethyl - 4 - [2 - (2 - methyl - 1,3 - dioxolanyl)] - 5,5 - dimethyl oxolane.

NMR

δ0.9 (3H, tW, 1.2 (9H, M), 1.3—1.5 (5H, M) 3.3 (2H, d), 3.8 (4H, br s), 4.4 (2H, s), 7.2 (5H, s).

Example 2

2-Ethyl-2-benzyloxymethyl-4-methylcarbonyl-5,5-dimethyl oxolane

The oxolane prepared in Example 1(e) (1 g), p-toluene sulphonic acid (0.25 g) and acetone solvent (10 ml) were combined and left standing at room temperature for 2 hours. The mixture was then washed with sodium bicarbonate solution and extracted 2 times with ether. The ether extracts were combined and dried using magnesium sulphate. The ether solvent was evaporated off and the residue was chromatographed in a silica column using a 4% acetone/petrol eluant. Evaporation of the solvent afforded 0.66 g corresponding to a 75.9% yield of 2 - ethyl - 2 - benzyloxymethyl - 4 - methylcarbonyl - 5,5 - dimethyl oxolane.

NMR

δ0.9 (6H, M), 1.5 (3H, s), 2.1 (3H, s), 1.3—2.5 (5H, M), 3.3 (2H, d), 4.5 (2H, s), 7.2 (5H, s).

Example 3

2-Ethyl-2-benzyloxymethyl-4-(1-hydroxyethyl)-5,5-dimethyl oxolane

The ketone of Example 2 (1 g) in diethylether (6 ml) was added slowly to a stirred mixture of lithium aluminium hydride (0.07 g) in diethyl ether (2 ml). The mixture was then refluxed with stirring for 1 hour. Subsequently the mixture was washed with water, 15% sodium hydroxide and water after which it was filtered through florasil and washed with ether. After drying with magnesium sulphate the ether solvent was evaporated to afford 0.89 g, corresponding to a 89% yield of 2 - ethyl - 2 - benzyloxymethyl - 4 - (1 - hydroxyethyl) - 5,5 - dimethyl oxolane.

NMR

δ0.9 (3H, t), 1—2.3 (14H, M), 3.3 (2H, br d), 3.5 (2H, br), 4.4 (2H, s), 7.2 (5H, s).

Example 4

2-Ethyl-2-benzyloxymethyl-4-(1-oximidoethyl)-5,5-dimethyl oxolane

The ketone of Example 2 (1 g) was combined with hydroxylamine . HCl (0.27 g) in pyridine (30 ml) and

the mixture was refluxed for 1 hour. Subsequently the mixture was washed with water and extracted with ether. The ether extract was washed with brine and dried using magnesium sulphate. Evaporation of the ether solvent afforded 0.84 g of 2 - ethyl - 2 - benzyloxymethyl - 4 - (1 - oximidoethyl) - 5,5 - dimethyl oxolane.

NMR

δ0.7—2.5 (14H, M), 1.8 (3H, s), 3.3 (2H, M), 4.4 (2H, br s), 7.2 (5H, s), 8.8 (1H, br).

Example 5

2-Ethyl-2-benzyloxymethyl-4-(1-hydroxy-1-propyn-1-ylethyl)-5,5-dimethyl oxolane

Magnesium (0.19 g) in ether solvent (10 ml) was mixed with bromoethane in ether (10 ml) and stirred until the magnesium had dissolved. Subsequently, propyne gas was bubbled through the mixture until all of the ethane had been removed. At this point, the ketone of Example 2 (1 g) in ether (10 ml) was added to the mixture and the mixture was refluxed for 2 hours. The reaction mixture was then washed with aqueous ammonium chloride and the resulting aqueous phase was washed twice with ether. The organic phases from the 3 washings were combined and washed with brine. After drying with magnesium sulphate the solvent was evaporated to yield 1.14 g corresponding to an 87.7% yield of 2 - ethyl - 2 - benzyloxymethyl - 4 - (1 - hydroxy - 1 - propyn - 1 - ylethyl) - 5,5 - dimethyl oxolane.

NMR

δ0.8—2.4 (20H, M), 3.3 (3H, M), 4.5 (2H, br s), 7.2 (5H, s).

Example 6

2-Ethyl-2-(2-fluorophenylmethoxymethyl)-4-[2-(2-methyl-1,3-dioxolanyl)]-5,5-dimethyl oxolane

The oxolane as prepared in Example 1(d) (1 g) in toluene (10 ml) was added slowly to a stirred mixture of sodium hydride in toluene (6 ml) under nitrogen. The mixture was refluxed for 1 hour at which time a solution of 2-fluorobenzyl chloride (0.77 g) in toluene (w ml) was added slowly. The resulting reaction mixture was refluxed for 18 hours after which it was washed with brine and extracted with ether. The organic phases were combined, washed with brine and dried using magnesium sulphate. The solvent was evaporated off and the residue was chromatographed using a silica column and 3% acetone/petrol as eluant. Evaporation of the solvent afforded 0.78 g corresponding to a 54.2% yield of 2 - ethyl - 2 - (2 - fluorophenyl)methoxymethyl - 4 - [2 - (2 - methyl - 1,3 - dioxolanyl)] - 5,5 - dimethyl oxolane.

NMR

δ0.9 (3H, t), 1—2.5 (14H, M), 3.3 (2H, d), 3.8 (4H, br s), 4.5 (2H, s), 7.1 (4H, M).

Examples 7—11

Other tetrahydrofuran derivatives

Using procedures similar to those described previously the following compounds according to the invention were prepared.

Example 7

2-Ethyl-2-(2-chlorophenylmethoxymethyl)-4-[2-(2-methyl-1,3-dioxolanyl)]-5,5-dimethyl oxolane

NMR

δ0.9 (3H, t), 1—2.5 (14H, M), 3.3 (2H, d), 3.8 (4H, br s), 4.6 (2H, s), 7.2 (4H, M).

Example 8

2-Ethyl-2-(2-pyridylmethoxymethyl)-4-[2-(2-methyl-1,3-dioxolanyl)]-5,5-dimethyl oxolane

NMR

δ0.9 (3H, t), 1—2.5 (14H, M), 3.3 (2H, d), 3.8 (4H, br s), 4.7 (2H, s), 6.9—7.6 (3H, M), 8.4 (1H, M).

Example 9

2-Ethyl-2-(2-fluorophenylmethylmethoxy)-4-methylcarbonyl-5,5-dimethyl oxolane

NMR

δ0.9—1.4 (6H, M), 1.6 (3H, s), 1.6—3.3 (3H, M), 2.2 (3H, s), 3.4 (2H, d), 4.6 (2H, s), 7.1 (4H, M).

Example 10

2-Ethyl-2-(2-chlorophenylmethylmethoxy)-4-methylcarbonyl-5,5-dimethyl oxolane

NMR

δ0.9—1.4 (H, M), 1.6 (3H, s), 1.6—3.3 (3H, M), 2.2 (3H, s), 3.4 (2H, d), 4.6 (2H, s), 7.2 (4H, M).

Example 11

2-Ethyl-2-(2-pyridylmethoxymethyl)-4-methylcarbonyl-5,5-dimethyl oxolane

NMR

δ0.9—1.4 (6H, M), 1.6 (3H, s), 1.6—3.3 (3H, M), 2.4 (3H, s), 3.4 (2H, d), 4.6 (2H, s), 7.5 (3H, M), 8.7 (1H, M).

Example 12

Herbicidal activity

To evaluate their herbicidal activity, compounds according to the invention were tested using as a representative range of plants: maize, *Zea mays* (MZ); rice, *Oryza sativa* (R); barnyard grass, *Echinochloa crusgalli* (BG); oat, *Avena sativa* (O); linseed, *Linum usitatissimum* (L); mustard, *Sinapsis alba* (M); sugar beet, *Beta vulgaris* (SB) and soya bean, *Glycine max* (S).

The tests fall into two categories, pre-emergence and post-emergence. The pre-emergence tests involved spraying a liquid formulation of the compound onto the soil in which the seeds of the plant species mentioned above had recently been sown. The post-emergence tests involved two types of test, viz., soil drench and foliar spray tests. In the soil drench tests the soil in which the seedling plants of the above species were growing, was drenched with a liquid formulation containing a compound of the invention, and in the foliar spray tests the seedling plants were sprayed with such a formulation.

The soil used in the tests was a prepared horticultural loam.

The formulations used in the tests were prepared by diluting with water, solutions of the test compounds in acetone containing 0.4% by weight of an alkylphenol/ethylene oxide condensate available under the trade name Triton X-155. The acetone solutions were diluted with water and the resulting formulations applied at dosage levels corresponding to 5 kg and/or 1 kg of active material per hectare in a volume equivalent to 650 litres per hectare in the soil spray and foliar spray tests, and at a dosage level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually eleven days after spraying the foliage and drenching the soil and twelve days after spraying the soil, and were recorded on a 0—9 scale. A rating 0 indicates growth as untreated control, a rating 9 indicates death. An increase of 1 unit on the linear scale approximates to a 10% increase in the level of effect.

The results of the tests are set out in Table I below.

TABLE I

| Compound of Example No. | Soil drench 10 kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 1 | 9 | 7 | 9 | 8 | 0 | 0 | 0 | 0 | 5 | 8 | 3 | 8 | 5 | 4 | 4 | 3 | 6 | 9 | 8 | 9 | 5 | 4 | 2 | 3 | 0 |
| | | | | | | | | | 1 | 4 | 0 | 7 | 2 | 0 | 2 | 0 | 3 | 9 | 5 | 9 | 2 | 3 | 0 | 0 | 0 |
| 2 | 8 | 8 | 9 | 8 | 3 | 4 | 5 | 3 | 5 | 7 | 4 | 8 | 7 | 4 | 5 | 5 | 5 | 9 | 8 | 9 | 6 | 4 | 0 | 5 | 2 |
| | | | | | | | | | 1 | 6 | 2 | 7 | 0 | 3 | 3 | 4 | 4 | 8 | 8 | 9 | 4 | 3 | 0 | 3 | 0 |
| 3 | 7 | 7 | 8 | 7 | 0 | 5 | 5 | 4 | 5 | 7 | 5 | 9 | 7 | 5 | 4 | 4 | 4 | 8 | — | 9 | 5 | 3 | 0 | 0 | 4 |
| | | | | | | | | | 1 | 6 | 3 | 7 | 4 | 2 | 0 | 2 | 2 | 6 | — | 9 | 5 | 0 | 0 | 0 | 0 |
| 4 | 8 | 7 | 8 | 7 | 4 | 3 | 0 | 0 | 5 | 7 | 5 | 7 | 3 | 3 | 3 | 0 | 3 | 7 | 7 | 9 | 6 | 5 | 0 | 3 | 3 |
| | | | | | | | | | 1 | 5 | 0 | 4 | 0 | 2 | 0 | 0 | 2 | 6 | 7 | 8 | 3 | 2 | 0 | 0 | 2 |
| 5 | — | — | — | — | — | — | — | — | 5 | — | — | — | — | — | — | — | — | — | — | — | — | — | — | — | — |
| | | | | | | | | | 1 | 5 | 4 | 6 | 4 | 0 | 0 | 0 | 4 | 6 | 5 | 8 | 4 | 0 | 0 | 0 | 0 |
| 6 | 8 | 7 | 7 | 8 | 0 | 0 | 0 | 0 | 5 | 7 | 5 | 6 | 6 | 3 | 2 | 2 | 5 | 8 | 8 | 9 | 7 | 5 | 4 | 3 | 5 |
| | | | | | | | | | 1 | 5 | 0 | 6 | 2 | 0 | 0 | 0 | 3 | 6 | 5 | 8 | 5 | 3 | 2 | 2 | 3 |
| 7 | 8 | 7 | 7 | 6 | 0 | 0 | 0 | 0 | 5 | — | — | — | — | — | — | — | — | — | — | — | — | — | — | — | — |
| | | | | | | | | | 1 | 2 | 0 | 5 | 1 | 4 | 1 | 5 | 4 | 5 | 0 | 9 | 5 | 5 | 1 | 3 | 0 |
| 8 | 9 | 7 | 8 | 8 | 4 | 0 | 0 | 0 | 5 | 6 | 6 | 7 | 7 | 4 | 3 | 3 | 5 | 7 | 8 | 8 | 7 | 5 | 2 | 3 | 3 |
| | | | | | | | | | 1 | 5 | 5 | 6 | 6 | 2 | 1 | 0 | 4 | 7 | 7 | 8 | 6 | 3 | 0 | 0 | 0 |
| 9 | 9 | 8 | 8 | 8 | 2 | 5 | 4 | 3 | 5 | 6 | 5 | 7 | 6 | 4 | 3 | 5 | 5 | 8 | 8 | 9 | 7 | 5 | 4 | 2 | 3 |
| | | | | | | | | | 1 | 6 | 4 | 6 | 3 | 2 | 2 | 1 | 3 | 7 | 7 | 9 | 7 | 4 | 2 | 0 | 3 |
| 10 | 8 | 8 | 8 | 7 | 2 | 5 | 3 | 2 | 5 | 6 | 5 | 7 | 5 | 5 | 5 | 4 | 4 | 7 | 7 | 9 | 5 | 5 | 4 | 4 | 3 |
| | | | | | | | | | 1 | 5 | 3 | 6 | 2 | 1 | 2 | 2 | 3 | 6 | 5 | 9 | 3 | 4 | 1 | 3 | 0 |
| 11 | 8 | 7 | 7 | 7 | 0 | 0 | 0 | 0 | 5 | 6 | 6 | 5 | 5 | 0 | 0 | 1 | 2 | 7 | 8 | 8 | 8 | 0 | 0 | 0 | 0 |
| | | | | | | | | | 1 | 4 | 5 | 4 | 5 | 0 | 0 | 0 | 0 | 6 | 8 | 8 | 7 | 0 | 0 | 0 | 0 |

**Claims**

1. A tetrahydrofuran derivative of the general formula:—

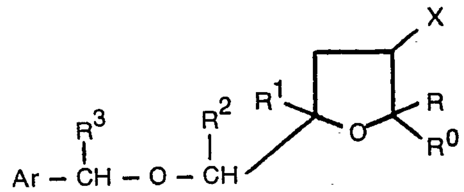

(I)

wherein each of $R^0$, R, $R^1$, $R^2$ and $R^3$ independently represents a hydrogen atom or an alkyl group of up to 6 carbon atoms, or $R^0$ and R together represent an alkylene group of up to 6 carbon atoms; X represents one of the groups

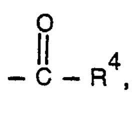, 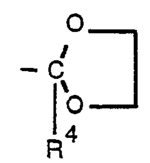, 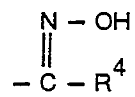 or 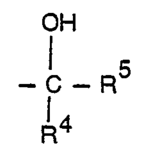

wherein $R^4$ represents a hydrogen atom, an alkyl group of up to 6 carbon atoms optionally substituted by one or more of the same or different substituents selected from halogen atoms, alkoxy, alkylthio, cyano, carboxy, alkoxycarbonyl, amino and cycloalkyl groups, optionally substituted phenyl and phenoxy groups, and groups of the formula OA in which A represents a hydrogen atom or an acyl group derived from a carboxylic or a substituted carbamic acid, or represents a phenyl group optionally substituted by one or more of the same or different substituents chosen from halogen atoms, hydroxy, amino, nitro and cyano groups and optionally substituted alkyl, alkenyl, alkynyl, alkoxy, alkylthio, alkoxycarbonyl, aryl and aryloxy groups; $R^5$ represents a hydrogen atom, or an alkyl, alkenyl or alkynyl group of up to 6 carbon atoms; and Ar represents a pyridyl or phenyl group, optionally substituted by one of substituents specified above in respect of a phenyl group $R^4$.

2. A compound as claimed in claim 1 in which each of $R^0$, and R is the same and each represents an alkyl group having up to 4 carbon atoms.

3. A compound as claimed in claim 1 or 2 in which $R^1$ represents an alkyl group having up to 4 carbon atoms.

4. A compound as claimed in any one of the preceding claims in which $R^2$ and $R^3$ each represents a hydrogen atom.

5. A compound as claimed in any one of the preceding claims wherein $R^4$ represents an alkyl group having up to 4 carbon atoms and $R^5$ represents a hydrogen atom or an alkyl or alkynyl group having up to 4 carbon atoms.

6. A compound as claimed in any one of the preceding claims in which Ar represents a pyridyl group or a phenyl group optionally substituted with a fluorine and/or chlorine atom.

7. A process for the preparation of a compound of the general formula I as defined in claim 1 which comprises converting a compound of the general formula:—

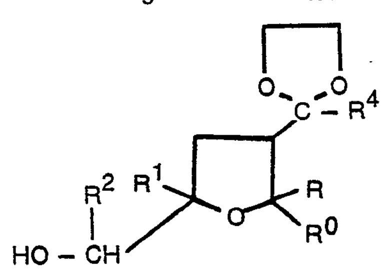

(II)

in which $R^0$, R, $R^1$, $R^2$ and $R^4$ have the meanings as defined in claim 1, into an alkali metal or alkaline earth metal salt thereof, and reacting the salt with a compound of the general formula:—

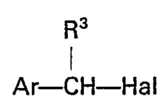

(III)

in which Hal represents a halogen atom and $R^3$ and Ar have the meanings as defined in claim 1; and optionally converting the resulting compound of the general formula I in which X represents a

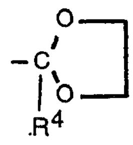

group into any other required compound of the general formula I.

8. A process as claimed in claim 7 in which the starting compound of formula II has been prepared by subjecting a compound of the general formula:—

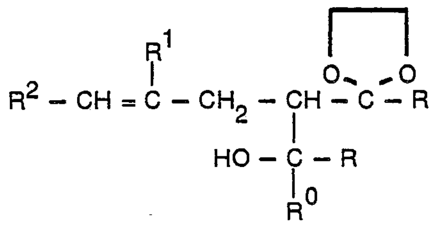

(IV)

in which $R^0$, R, $R^1$, $R^2$ and $R^4$ have the meanings as defined in claim 1, to ring closure by reaction with a electrophilic epoxidizing agent.

9. A herbicidal composition which comprises a compound as claimed in any one of claims 1 to 6, together with a carrier.

10. A composition as claimed in claim 9, which comprises at least two carriers, at least one of which is a surface-active agent.

11. A method of combating undesired plant growth at a locus, which comprises treating the locus with a compound as claimed in any one of claims 1 to 6 or a composition as claimed in claim 9 or 10.

**Patentansprüche**

1. Ein Tetrahydrofuran-Derivat der allgemeinen Formel:

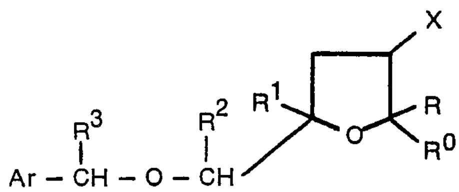

(I)

in welcher jede der Gruppen $R^0$, R, $R^1$, $R^2$ und $R^3$ unabhängig ein Wasserstoffatom oder eine Alkylgruppe mit bis zu 6 Kohlenstoffatomen darstellt, oder $R^0$ und R zusammen eine Alkylengruppe mit bis zu 6 Kohlenstoffatomen darstellen; X eine der Gruppen

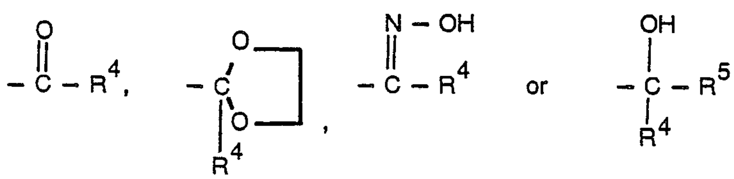

, , or

bedeutet, wobei $R^4$ ein Wasserstoffatom, eine Alkylgruppe mit bis zu 6 Kohlenstoffatomen darstellt, gegebenenfalls substituiert durch einen oder mehrere gleiche oder verschiedene Substituenten ausgewählt aus Halogenatomen, Alkoxy-, Alkylthio-, Cyano-, Carboxy-, Alkoxycarbonyl-, Amino- und Cycloalkylgruppen, gegebenenfalls substituierten Phenyl- und Phenoxygruppen und Gruppen der Formel OA, in welcher A ein Wasserstoffatom oder eine Acylgruppe abgeleitet, von einer Carbonsäure oder einer substituierten Carbamidsäure, oder eine Phenylgruppe, gegebenenfalls substituiert durch eine oder mehrere gleiche oder verschiedene Substituenten, ausgewählt aus Halogenatomen, Hydroxy-, Amino-, Nitro- und Cyanogruppen und gegebenenfalls substituierten Alkyl-, Alkenyl- Alkynyl-, Alkoxy-, Alkylthio-, Alkoxycarbonyl-, Aryl- und Aryloxygruppen, darstellt; $R^5$ ein Wasserstoffatom oder eine Alkyl-, Alkenyl- oder Alkynylgruppe mit bis zu 6 Kohlenstoffatomen bedeutet; und Ar eine Pyridyl- oder Phenylgruppe, gegebenenfalls substituiert durch eine der oben in bezug auf eine Phenylgruppe $R^4$ genannten Substituenten darstellt.

2. Eine Verbindung wie in Anspruch 1 beansprucht, in welcher die Gruppen $R^0$ und R jeweils gleich sind und jeweils eine Alkylgruppe mit bis zu 4 Kohlenstoffatomen darstellen.

3. Eine Verbindung wie in Anspruch 1 oder 2 beansprucht, in welcher $R^1$ eine Alkylgruppe mit bis zu 4 Kohlenstoffatomen darstellt.

4. Eine Verbindung wie in einem der vorstehenden Ansprüche beansprucht, in welcher $R^2$ und $R^3$ jeweils ein Wasserstoff bedeuten.

5. Eine Verbindung wie in einem der vorstehenden Ansprüche beansprucht, in welcher $R^4$ eine Alkylgruppe mit bis zu 4 Kohlenstoffatomen und $R^5$ ein Wasserstoffatom oder eine Alkyl- oder Alkynylgruppe mit bis zu 4 Kohlenstoffatomen darstellt.

6. Eine Verbindung wie in einem der vorstehenden Ansprüche beansprucht, in welcher Ar eine Pyridylgruppe oder eine Phenylgruppe, gegebenenfalls substituiert mit einem Fluor und/oder Chloratom, bedeutet.

7. Ein Verfahren zur Herstellung einer Verbindung der allgemeinen Formel I wie in Anspruch 1 definiert, welches die Umwandlung einer Verbindung der allgemeinen Formel

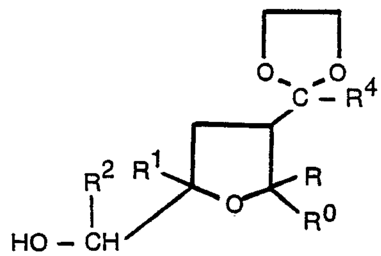

(II)

in welcher $R^0$, R, $R^1$, $R^2$ und $R^4$ die in Anspruch 1 angegebenen Bedeutungen haben, in ein Alkalimetall- oder Erdalkalimetallsalz derselben, und die Umsetzung des Salzes mit einer Verbindung der allgemeinen Formel

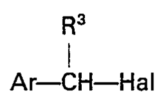

(III)

in welcher Hal ein Halogenatom bedeutet und $R^3$ und Ar die in Anspruch 1 angegebenen Bedeutungen haben; und gegebenenfalls die Umwandlung der daraus resultierenden Verbindung der allgemeinen Formel I, in welcher X eine Gruppe

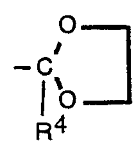

darstellt, in eine andere gewünschte Verbindung der allgemeinen Formel I, umfaßt.

8. Ein Verfahren wie in Anspruch 7 beansprucht, in welchem die Ausgangsverbindung der Formel II hergestellt wurde, indem eine Verbindung der allgemeinen Formel

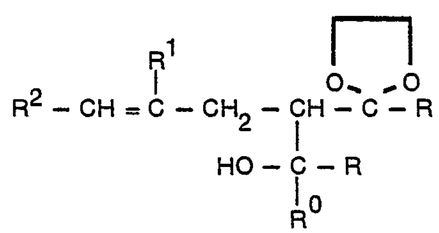

(IV)

in welcher $R^0$, R, $R^1$, $R^2$ und $R^4$ die in Anspruch 1 angegebenen Bedeutungen haben, durch Umsetzung mit einem elektrophilen Epoxidationsmittel einer Ringschlußreaktion unterworfen wurde.

9. Eine herbizide Zusammensetzung, welche eine Verbindung, wie in einem der Ansprüche 1 bis 6 beansprucht, zusammen mit einem Träger umfaßt.

10. Eine Zusammensetzung wie in Anspruch 9 beansprucht, welche mindestens 2 Träger umfaßt, wovon mindestens einer eine oberflächenaktive Substanz ist.

11. Eine Methode zur lokalen Bekämpfung von unerwünchstem Pflanzenwuchs, welche die Behandlung der betreffenden Stelle mit einer Verbindung, wie in einem der Ansprüche 1 bis 6 beansprucht, oder einer Zusammensetzung, wie in Anspruch 9 oder 10 beansprucht, umfaßt.

**Revendications**

1. Un dérivé du tétrahydrofuranne de la formule générale:

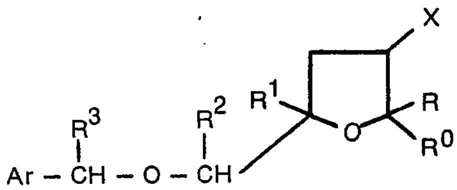

(I)

dans laquelle $R^0$, R, $R^1$, $R^2$ et $R^3$ représentent chacun indépendamment un atome d'hydrogène ou un groupe alcoyle de jusqu'à 6 atomes de carbone, ou $R^0$ et R représentent ensemble un groupe alcoylène de jusqu'à 6 atomes de carbone; X représente un des groupes

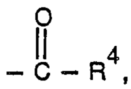 , 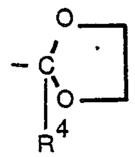 , 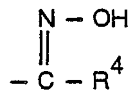 or 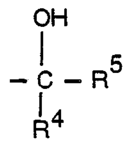

où $R^4$ représente un atome d'hydrogène, un groupe alcoyle de jusqu'à 6 atomes de carbone éventuellement substitué par un ou plusieurs substituants identiques ou différents choisis parmi des atomes d'halogènes, des groupes alcoxy, alcoylthio, cyano, carboxy, alcoxycarbonyle, amino et cycloalcoyle, des groupes phényle et phénoxy éventuellement substitués et des groupes de la formule OA où A représente un atome d'hydrogène ou un groupe acyle dérivé d'un acide carboxylique ou d'un acide carbamique substitué, ou représente un groupe phényle substitué par un ou plusieurs substituants identiques ou différents choisis parmi des atomes d'halogènes, des groupes hydroxy, amino, nitro et cyano et des groupes alcoyle, alcényle, alcynyle, alcoxy, alcoylthio, alcoxycarbonyle, aryle et aryloxy éventuellement substitués; $R^5$ représente un atome d'hydrogène ou un groupe alcoyle, alcényle ou alcynyle de jusqu'à 6 atomes de carbone; et Ar représente un groupe pyridyle ou phényle, éventuellement substitué par un des substituants spécifiés ci-dessus à propos d'un groupe phényle $R^4$.

2. Un composé selon la revendication 1, dans lequel $R^0$ et R sont identiques et représentent chacun un groupe alcoyle ayant jusqu'à 4 atomes de carbone.

3. Un composé selon la revendication 1 ou 2, dans lequel $R^1$ représente un groupe alcoyle ayant jusqu'à 4 atomes de carbone.

4. Un composé selon l'une quelconque des revendications précédentes, dans lequel $R^2$ et $R^3$ représentent chacun un atome d'hydrogène.

5. Un composé selon l'une quelconque des revendications précédentes, dans lequel $R^4$ représente un groupe alcoyle ayant jusqu'à 4 atomes de carbone et $R^5$ représente un atome d'hydrogène ou un groupe alcoyle ou alcynyle ayant jusqu'à 4 atomes de carbone.

6. Un composé selon l'une quelconque des revendications précédentes, dans lequel Ar représente un groupe pyridyle ou un groupe phényle éventuellement substitué par un atome de fluor et/ou de chlore.

7. Un procédé pour la préparation d'un composé de la formule générale I tel que défini dans la revendication 1, qui comprend la transformation d'un composé de la formule générale:

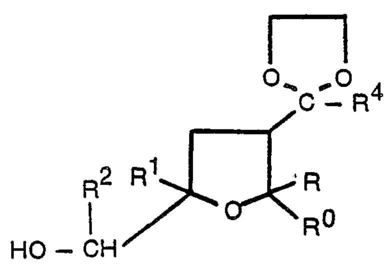

(II)

dans laquelle $R^0$, R, $R^1$, $R^2$ et $R^4$ ont les significations définies dans la revendication 1, en un sel de métal alcalin ou de métal alcalino-terreux de ce composé, et la réaction du sel avec un composé de la formule générale:

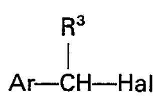

(III)

dans laquelle Hal représente un atome d'halogène et $R^2$ et Ar ont les significations définies dans la revendication 1; et éventuellement la conversion du composé résultant de la formule générale I où X représente un groupe

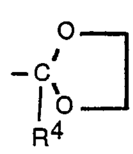

en un autre composé désiré quelconque de la formule générale I.

8. Un procédé selon la revendication 7, dans lequel on a préparé le composé de départ de formule II en soumettant un composé de la formule générale:

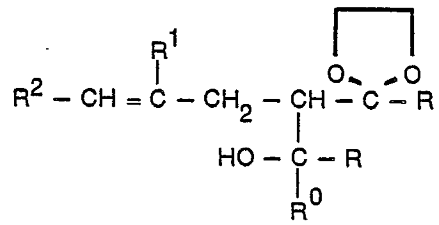

(IV)

dans laquelle $R^0$, R, $R^1$, $R^2$ et $R^4$ ont les significations définies dans la revendication 1, à une cyclisation par réaction avec un agent d'époxydation électrophile.

9. Une composition herbicide qui comprend un composé selon l'une quelconque des revendications 1 à 6 en même temps qu'un véhicule.

10. Une composition selon la revendication 9, qui comprend au moins deux véhicules, dont l'un au moins est un agent tensio-actif.

11. Un procédé de lutte contre la croissance de plantes indésirables en un lieu, qui comprend le traitement du lieu par un composé selon l'une quelconque des revendications 1 à 6 ou par une compositions selon la revendication 9 ou 10.